# EUROPEAN PATENT SPECIFICATION

(11) **EP 3 062 850 B1**
(45) Date of publication and mention of the grant of the patent: **26.03.2025**
(21) Application number: 14859162.1
(22) Date of filing: 30.10.2014
(51) Int. Cl.: A61M 5/32, A61M 5/24

(54) **METHOD AND DEVICE FOR INCREASING THE SAFETY IN CONJUNCTION WITH THE REMOVAL OF A CANNULA FROM A SYRINGE**
VERFAHREN UND VORRICHTUNG ZUR ERHÖHUNG DER SICHERHEIT IN VERBINDUNG MIT DER ABNAHME EINER KANÜLE VON EINER SPRITZE
PROCÉDÉ ET DISPOSITIF PERMETTANT D'AMÉLIORER LA SÉCURITÉ LORS DU RETRAIT DE LA CANULE D'UNE SERINGUE

(30) Priority: 30.10.2013 SE 1300677
(43) Date of publication of application: 07.09.2016
(73) Proprietor: Danderyds Snickeri AB, 187 66 Täby (SE)
(72) Inventor: HVERÉN, Lennart, S-182 34 Danderyd (SE)
(74) Representative: Basck Limited
(86) International application number: PCT/SE2014/000128
(87) International publication number: WO 2015/065259

(56) References cited:
- EP-A1- 1 655 047
- EP-A1- 2 529 777
- EP-A2- 2 420 279
- WO-A1-03/047664
- WO-A1-2011/139212
- US-A- 5 997 513
- US-A1- 2006 036 216
- US-A1- 2010 042 053

## Description

### Technical area of the invention

The present invention relates to a method for increasing the safety when removing a cannula from a syringe, wherein the cannula is removed from the syringe by a separation mechanism.

### Prior art

A cannula remover that comprises a separation mechanism for separating a cannula from a syringe is known from EP 2 420 279. In order to perform a separation of the cannula from the syringe, the cannula firmly threaded on the syringe is introduced into a receiving part of the cannula remover and an activation of the separation mechanism takes place by a manual rotation of the receiving part. The authorities have stated that there is a risk that the user can puncture himself with the cannula when the cannula remover is being used to remove the cannula from the syringe.

### GOALS AND SPECIAL FEATURES OF THE INVENTION

A primary goal of the present invention is to indicate a safety arrangement integrated with the syringe that reduces the risk of an operator puncturing himself.

Another goal of the present invention is that the safety arrangement is user-friendly.

Another goal of the present invention is that the safety arrangement is economical.

Another goal of the present invention is that the arrangement is preferably a single-use type, as a result of which the hygiene is extremely high.

At least the primary goal of the present invention is realized by a method and a device that achieve the characteristics indicated in the following independent claims. The invention is as defined in independent claims 1 and 3. Preferred embodiments of the invention are defined in the dependent claims.

### SHORT DESCRIPTION OF THE DRAWINGS

A preferred embodiment of the invention is described below with reference made to the attached drawings, in which:
Fig. 1A shows a first perspective view of a tube and a casing that are part of the device in accordance with the present invention;
Fig. 1 B shows a second perspective view of a tube and a casing that are part of the device in accordance with the present invention;
Fig. 2A shows a perspective view of the device according to the present invention, that is, when the casing is mounted on the tube;
Fig. 2B shows a section along IIB-IIB in fig. 2F;
Fig. 2C shows an enlarged detail of the part surrounded by the circle IIC in fig. 2B;
Fig. 2D shows a side view of the device according to the present invention, wherein a part of the casing is cut away;
Fig. 2E shows an enlarged detail of the part surrounded by the circle HE in fig. 2D;
Fig. 2F shows a section along IIF-IIF in fig. 2D;
Fig. 3A shows a side view of the device according to the present invention when it is mounted on a syringe;
Fig. 3B shows a section along NIB-NIB in fig. 3D;
Fig. 3C shows an enlarged detail of the part surrounded by the circle IIIC-IIIC in fig. 3B;
Fig. 3D shows a section along IND-IND in fig. 3A;
Fig. 4A shows a side view of the device according to the present invention when it is mounted on a syringe, wherein the device surrounds a cannula in the syringe;
Fig. 4B shows a section along IVB-IVB in fig. 4D;
Fig. 4C shows an enlarged detail of the part surrounded by the circle IVC-IVC in fig. 4B;
Fig. 4D shows a section along IVD-IVD in fig. 4A; Fig. 5A shows a side view of the device according to the present invention, wherein the device is mounted on a syringe and inserted in a holder component in a cannula remover;
Fig. 5B shows a section along VB-VB in fig. 5D;
Fig. 5C shows an enlarged detail of the part surrounded by the circle VC-VC in fig. 5B;
Fig. 5D shows a section along VD-VD in fig. 5A;
Fig. 6A shows a side view of the device according to the present invention, wherein the device is mounted on a syringe and inserted in a holding component in a cannula remover, and the syringe was shifted downward in comparison to fig. 5A;
Fig. 6B shows a section along VIB-VIB in fig. 6D;
Fig. 6C shows an enlarged detail of the part surrounded by the circle VIC-VIC in fig. 6B; and
Fig. 6D shows a section along VID-VID in fig. 6A.

### DETAILED DESCRIPTION OF A PREFERRED EMBODIMENT OF THE INVENTION

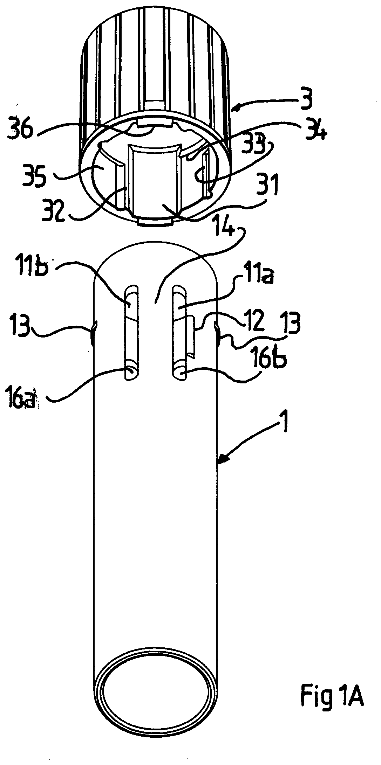

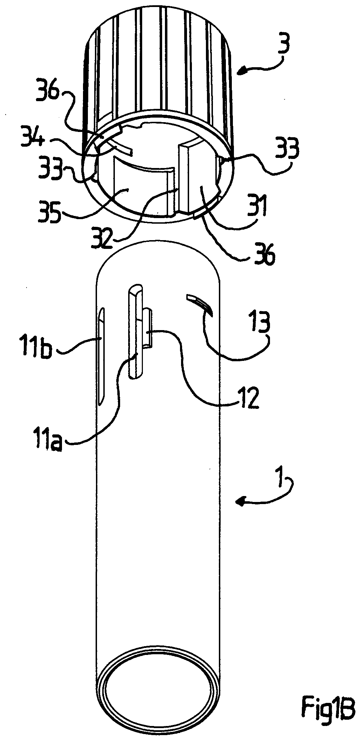

As is apparent from fig. 1A and 1 B, the device comprises, according to the present invention, a tube 1 and an annular casing 3 that can be mounted on one end of the tube 1. The tube 1 has a length that is a multiple of the length of the casing 3. In the preferred embodiment shown the tube 1 has a length that is approximately four times the length of the casing 3. When the casing 3 is mounted on the tube 1, the tube 1 and the casing 3 can rotate relative to one another around a common longitudinally running central axis.

In the embodiment shown the tube 1 has a circular cross section and its inside diameter is adapted to the outside diameter of the syringe S that the tube 1 is to be mounted on. In the embodiment shown the casing 3 also generally has a circular cross section and an inside diameter that is adapted to the outside diameter of the tube 1 in the area of the end of the tube 1 that the casing is to be mounted on. More precisely, the diameters are adapted in such a manner to one another that there is a sliding fit between the inside diameter of the casing 3 and the outside diameter of the end of the tube 1 that the casing 3 is to be mounted on. Fig. 1A and 1B show perspective views of the tube 1 and the casing 3. Both the tube 1 and the casing 3 consist of two halves that are mirror images of each other, that is, an imaginary, longitudinally running plane of symmetry divides the tube 1 and the casing 3. As can be seen most clearly from fig. 1A, the tube 1 is provided with two diametrically disposed groups of parallel slots 11A, 1 1 B, 16A, 16B in the area of one end of the tube 1. The slots 1 1A, 1 1 B, 16A, 16B extend in the longitudinal direction of the tube 1 and have a length constituting only a lesser part of the length of the tube 1. In the embodiment shown the distance between the slots 11 A, 1 1 B respectively 16A, 16B forming a group is less than the length of the slots 11A, 11 B, 16A, 16B. A flexible strip -like part 14 of tube 1 is defined between adjacent slots 1 A, 1 B respectively 16A, 6B, wherein this part has an extension in the longitudinal direction of the tube 1 that mainly coincides with the length of the slots 1 1 A, 11 B, 16A, 16B. An external first ridge 12 is disposed following the one slot 11A, 16A in each group, which ridge has a length according to the embodiment shown, that is less than the length of the associated slot 11 A, 16A.

The tube 1 also comprises two external, diametrically placed heels 13 that have a certain extension in the circumferential direction of the tube. The extension of the heels 13 in the circumferential direction constitutes only a lesser part of the entire circumference of the tube 1.

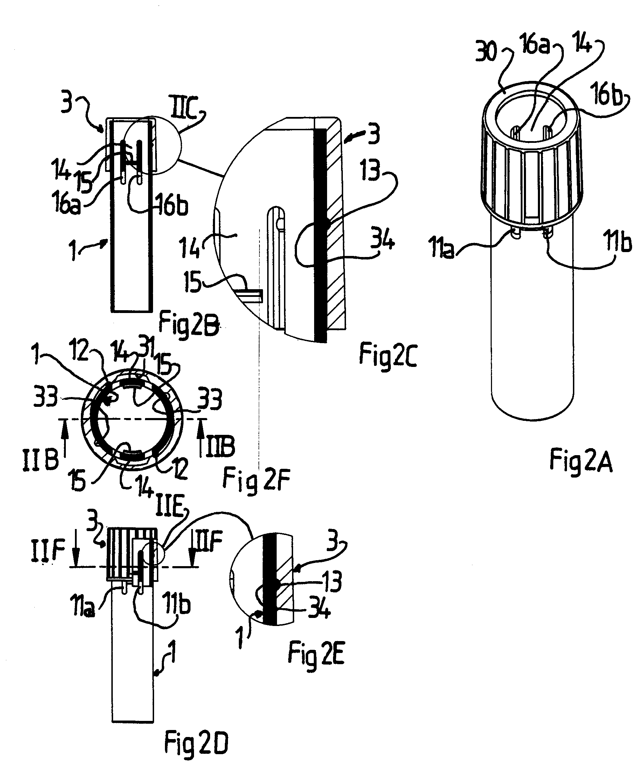

Fig. 2B shows an internal part, that is, one half of tube 1, wherein the not-shown, internal part of the tube 1 has a corresponding shape, that is, the tube 1 consists of two halves that are the mirror image of one another. Fig. 2B shows the two slots 16A, 16B. Fig. 2B and 2C also show an internal heel 15 that is located on the flexible part 14, wherein the heel 5 extends mainly between the slots 16A, 16B in the circumferential direction of the tube 1. According to the embodiment shown the internal heel 15 is located approximately in the middle of the height of the flexible part 14.

Fig. 1A and 1 B show perspective views of internal parts of the casing 3, wherein the casing 3 therefore consists of two halves that are mirror images of one another. As is apparent from fig. 2A, the casing 3 comprises on its one end a col- lar 30 that forms a stop shoulder when the casing 3 is mounted on the one end of the tube 1.

According to the embodiment shown, the casing 3 is provided with two internal recesses 31 that are generally square and located diametrically in front of one another. The internal recesses 31 extend in the longitudinal/vertical direction of the casing 3 corresponding approximately to half the height of the casing 3. As concerns the extent of the internal recesses 31 in the circumferential direction of the casing 3, it constitutes only a lesser part of the internal circumference of the casing 3 and corresponds in the embodiment shown mainly to the distance in the circumferential direction of the tube 1 between adjacent slots 11 A and 11 B, respectively 16A, 16B. Each of the internal recesses 31 is limited on the one side by another ridge 32 that was formed in that the wall thickness of the casing 3 becomes narrower in the direction of the recesses 31 in the circumferential direction of the casing 3. The other ridges 32 extend in the longitudinal direction/vertical direction of the casing 3 and according to the embodiment shown the other ridges 32 have an extent in the longitudinal direction/vertical direction of the casing 3 that constitutes the greater part of the height of the recesses 31.

The casing 3 is provided with two internal first tracks 33 that extend in the longitudinal direction/vertical direction of the casing 3. According to the embodiment shown the length of the tracks 33 is somewhat shorter than the height of the associated recess 3.

As is most clearly apparent from fig. 1 B, the casing 3 comprises internal parts 35 with a varying wall thickness in the circumferential direction. These parts 35 are disposed following the recesses 31 and in the same part of the height of the casing 3. The parts 35 have the least wall thickness following the recesses 31 and, as was indicated above, this forms the other ridges 32. These parts 35 have an increasing wall thickness in the direction away from the other ridges 32 and end approximately halfway between another ridge 32 and an internal first track 33. The casing 3 is provided with two internal other tracks 34 that extend in the circumferential direction of the casing 3 and are located approximately at the half height of the casing 3. The length of the other tracks 34 is shorter than the circumferential distance between adjacent edges of the two diametrical recesses and according to the embodiment shown the length of the second track is approximately 2/3 of the circumference distance between adjacent edges of the diametrically located recesses 31.

Fig. 2A-2F show different views, sections and details of how the casing 3 is mounted on the tube 1 in a starting position. As is most clearly apparent from fig. 2F, the casing 3 is mounted on the tube 1 in such a manner that both recesses 31 in the casing 3 are located right in front of the flexible parts 14 of the tube 1. In order to facilitate the correct mounting of the casing 3 on the tube 1, the casing 3 is provided with tongues 36 right in front of the recesses 31. When the casing 3 is pushed entirely on the tube 1, the external heels 13 on the tube 1 engage with the other tracks 34 on the casing 3. This is shown most clearly in fig. 2C and 2E.

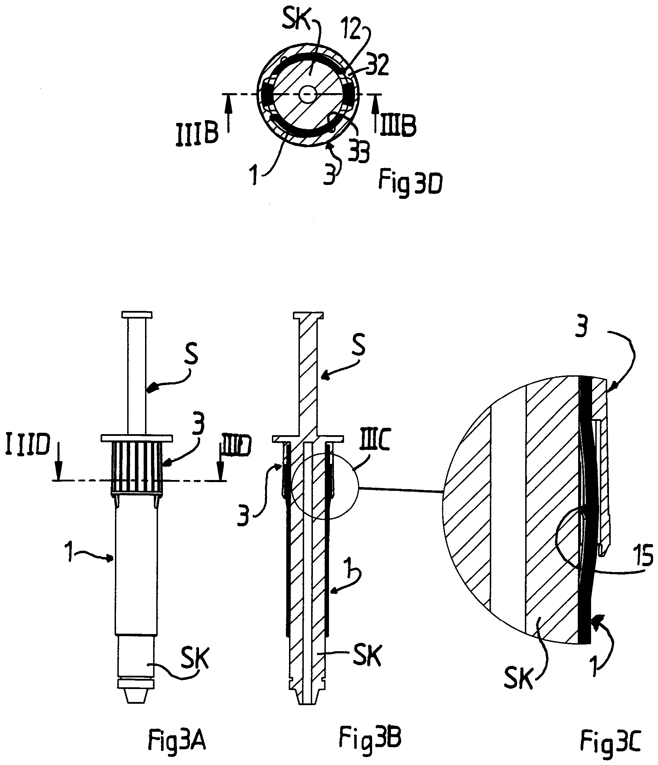

When the tube 1 and the casing 3 are in the assembled position shown in fig. 2A-2F, the tube 1 and the casing 3 are pushed onto an injection body SK in a syringe S. This is shown in fig. 3A-3D.

Before the tube 1 and the casing 3 are placed on the injection body SK, an ampule is mounted in a space in the injection body SK. This is not shown in fig. 3A-3D. As is apparent from fig. 3A and 3B, the casing 3 is preferably brought in contact with a flange FL on the syringe S.

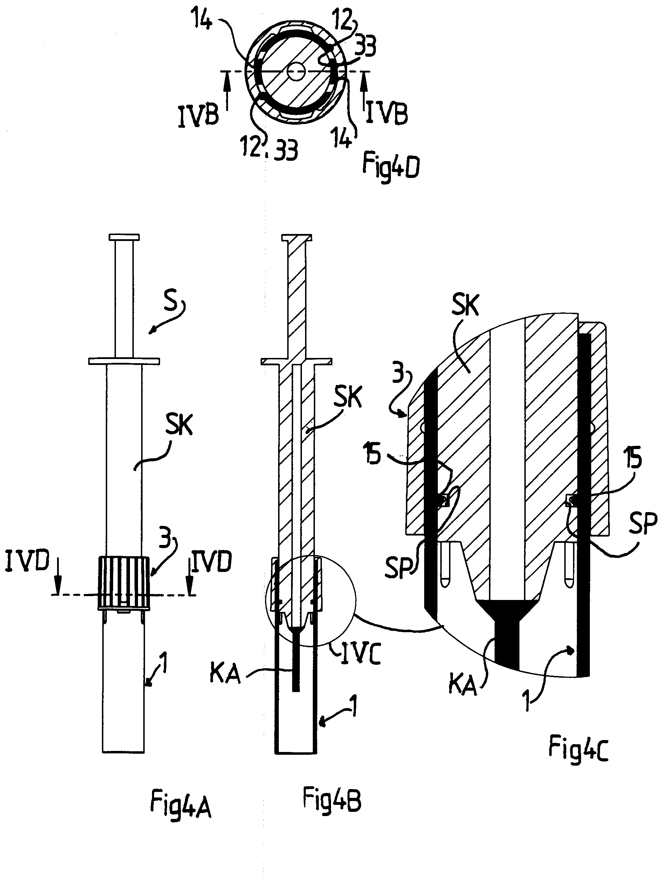

When the tube 1 and the casing 3 have been pushed onto the injection body SK, a cannula KA is mounted on the injection body SK, see fig. 4B. When the tube 1 and the casing 3 are located in the position shown in fig. 3A and 3B and a cannula KA is mounted on the injection body SK, an injection with the syringe S can take place, wherein a normal anesthetic is injected in the oral cavity of a patient who is at the dentist's.

When the injection has been completed, the tube 1 and the casing 3 are pushed into the position shown in fig. 4A and 4B where the internal heels 15 on the tube 1 are taken up in third track SP going around the front end of the injection body SK, that is, the end at which the cannula KA is mounted. During the dis- placement of the tube 1 and the casing 3 from the position shown in fig. 3A and 3B to the position shown in fig. 4A and 4B, the user notices when the correct position according to fig. 4A and 4B has been reached when the heels 15 click into the third track SP. In order to further anchor the tube 1 and the casing 3 on the injection body SK a mutual rotation of the tube 1 and the casing 3 takes place. A comparative study of fig. 2F and fig. 4D shows that the tube 1 has rotated 90° relative to the casing 3, wherein the rotation of the tube 1 took place counterclockwise while the casing 3 did not rotate, during which the external ridge 12 on the tube 1 clicked into the internal first track 33 on the casing. As an alternative to rotating the tube 1, the casing 3 can be rotated while the tube 1 is not rotated.

During the above-described mutual rotation between the tube 1 and the casing 3, in the position according to fig. 4D thicker wall parts of the casing 3 come to be located directly in front of the flexible parts 14 in the tube 1. These thicker wall parts come to exert a clamping action on the flexible parts 4, so that the latter press against the injection body SK. Since the internal heels 15 are located on the inside of the flexible parts 14, this clamping action presses the heels 15 into the third track SP in the injection body SK. As a consequence, a very good fixing of the tube 1 relative to the injection body SK takes place.

Syringe S with the tube 1 and the casing 3 is now ready to be used in a cannula remover, which will be schematically described below.

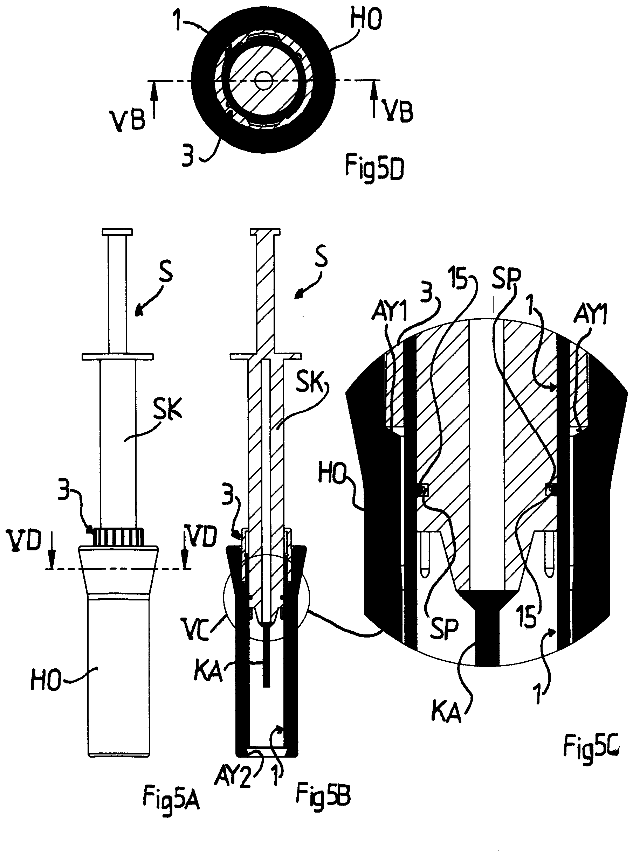

Fig. 5A-5D show a holding component HO in the form of a casing that forms a part of a cannula remover that is described in SE 535 606 C2. Refer to the indicated document for more information about how the removal of the cannula KA from the injection body SK takes place. As concerns the method and the device according to the present invention, only the holding component HO is of interest.

As is most clearly apparent from fig. 5B and 5C, the syringe with the tube 1 and the casing 3 is moved down into the holding component HO, wherein the end of the casing 3 that is pushed onto the tube 1 comes to rest against an internal first stop surface AY1 on the holding component HO. According to the embodiment shown, the internal first stop surface AY1 extends around the entire internal, cylindrical space of the holding component HO, that is, the internal stop surface AY1 is generally located in a plane that extends at a right angle to the longitudinal direction of the holding component HO. Fig. 5B and 5C show the contact of the casing 3 against the holding component HO. Fig. 5B also shows that a certain mutual displacement took place between the tube 1 and the casing 3, that is, the tube 1 shifted downwards by a stretch that corresponds approximately to one half the height of the casing 3. This mutual displacement between the tube 1 and the casing 3 is initiated in that the syringe S was shifted downward by a certain stretch by the operator, during which the engagement of the internal heels 15 with the track SP on the injection body SK entrains the tube 1 when the syringe S is shifted downward. Since the ridges 12 on the tube 1 are taken up into the first tracks 33 in the casing 3, the ridges 12 slide in the first tracks 33 during the displacement of the tube 1 relative to the casing 3.

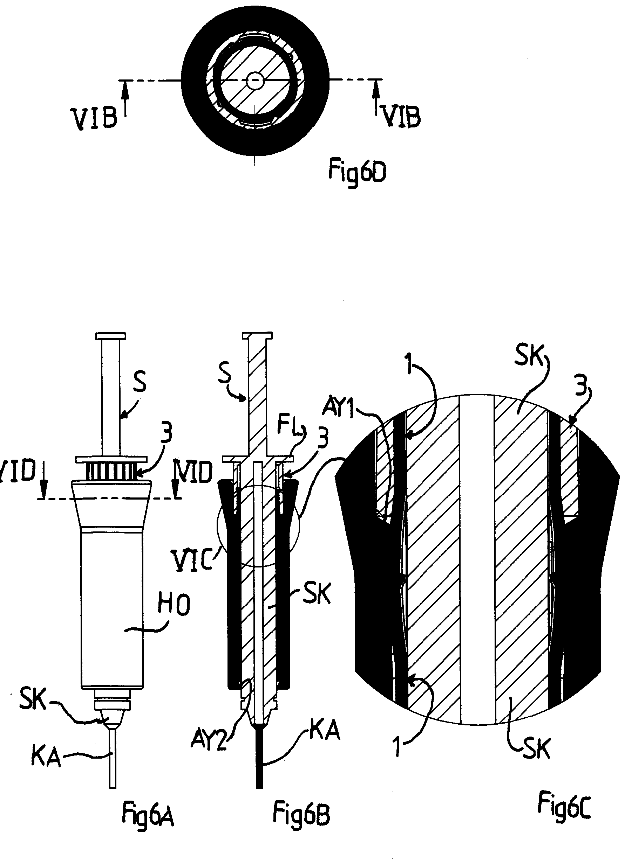

During the further downward displacement of the syringe S, see fig. 6A and 6B, the lower end of the tube 1 comes to rest against another stop surface AY2 on the holding component HO, which other stop surface AY2 is located on the lower end of the holding component HO. As a result, it is prevented that the lower end of the tube 1 is shifted downward past the lower end of the holding component HO.

Fig. 6A-6D show how the syringe S was shifted to a lower end position in the holding component HO, wherein a flange FL on the syringe S comes to rest against the upper end of the casing 3 when the lower end position of the syringe S has been reached. As is apparent from fig. 6A and 6B, cannula KA has now been shifted downward past the lower end of the holding component HO and the separation mechanism of the cannula remover can now carry out a separation of the cannula KA from the injection body SK.

The fit between the tube 1 and the injection body SK is now so tight that the tube 1 and the casing 3 also follow upward when the syringe S is drawn up out of the holding component HO.

## Claims

1. A device for increasing safety in conjunction with removal of a cannula (KA) from an injection body (SK) of a syringe, the device comprising:
- a tube (1) having a group of parallel longitudinal slots consisting of two diametrically disposed pairs of parallel slots (11A, 11B, 16A, 16B), wherein a flexible strip (14) is defined between each of two pairs of adjacent slots of the group of parallel longitudinal slots (11A, 11B, 16A, 16B);
- a casing (3) mounted on the tube (1) and provided with two internal recesses (31) extending in a longitudinal direction of the casing (3), wherein the casing (3) has a variable wall thickness in circumferential direction; and
- a holding component (HO) that is operable to receive the casing (3) along with the tube (1), wherein during downward displacement of the casing (3) and the tube (1) the casing (3) comes to rest against an internal first stop surface (AY1) of the holding component (HO) and during further downward displacement, the lower end of the tube (1) comes to rest against another stop surface (AY2) of the holding component (HO);
wherein the cannula (KA) is removable from the syringe (S) by a cannula removing separation mechanism,
**characterized in that** the tube (1) and the casing (3) are operable to rotate around a common longitudinally running central axis such that thicker wall parts of the casing (3) come to be located directly in front of the flexible strip (14) in the tube (1) and exert a clamping action on the flexible strip ( 14) for removal of the cannula (KA) from the syringe (S), wherein the clamping action presses an internal heel (15) located on the inside of the flexible strip of the tube (1) into a track (SP) going round the front end of the injection body (SK) in the injection body (SK), thus providing clamping of the tube (1) relative to the injection body (SK).

2. A device according to claim 1, **characterized in that** the casing (3) further comprises tracks (34) for mutually cooperating with the engagement components the at least one heel (13) of the tube (1).

3. A method of increasing safety in conjunction with removal of a cannula (KA) from a syringe by the device of claim 1, **characterized in that** the method comprises:
- mounting a casing (3) on a tube (1);
- pushing the casing (3) and the tube (1) onto an injection body (SK) of the syringe;
- mounting a cannula (KA) on the injection body (SK);
- pushing the casing (3) and the tube (1) towards a front end of the injection body (SK), such that the clamping action presses an internal heel (15) located on the inside of flexible strip of the tube (1), wherein the flexible strip (14) is defined between each of two pairs of adjacent slots of a group of parallel longitudinal slots consisting of two diametrically disposed pairs of parallel slots (11A, 11B, 16A, 16B),click into a track (SP) going round the front end of the injection body (SK);
- rotating the tube (1) with respect of casing (3) for anchoring the tube (1) and casing (3) on the injection body (SK) such that thickerwall parts of the casing (3) come to be located directly in front of the flexible strip (14) in the tube (1) and exert a clamping action on the flexible strip ( 14);
- moving the tube (1), the casing (3) and the syringe (S) into the holding unit (HO) of a cannula removing separation mechanism, such that during downward displacement of the casing (3) and the tube (1) the casing (3) comes to rest against an internal first stop surface (AY1) of the holding component (HO) and during further downward displacement, the lower end of the tube (1) comes to rest against another stop surface (AY2) of the holding component (HO); and
- removing the cannula from the injection body by a cannula removing separation mechanism.

4. A method according to claim 3, **characterized in that** the clamping action between the tube (1) and the injection body (SK) is achieved by a varying wall thickness of the casing (3).

## Patentansprüche

1. Vorrichtung zum Erhöhen einer Sicherheit im Zusammenhang mit einer Entfernung einer Kanüle (KA) aus einem Injektionskörper (SK) einer Spritze, die Vorrichtung umfassend:
- eine Röhre (1), die eine Gruppe paralleler Längsschlitze aufweist, die aus zwei diametral angeordneten Paaren paralleler Schlitze (11A, 11B, 16A, 16B) besteht, wobei zwischen jedem der zwei Paare angrenzender Schlitze der Gruppe paralleler Längsschlitze (11A, 11B, 16A, 16B) ein flexibler Streifen (14) definiert ist;
- eine Hülse (3), die auf der Röhre (1) montiert ist und mit zwei inneren Aussparungen (31) versehen ist, die sich in einer Längsrichtung der Hülse (3) erstrecken, wobei die Hülse (3) in Umfangsrichtung eine variable Wandstärke aufweist; und
- eine Haltekomponente (HO), die betriebsfähig ist, um die Hülse (3) zusammen mit der Röhre (1) aufzunehmen, wobei, während einer Abwärtsverschiebung der Hülse (3) und der Röhre (1), die Hülse (3) an einer inneren ersten Anschlagoberfläche (AY1) der Haltekomponente (HO) zum Stillstand kommt und, während einer weiteren Abwärtsverschiebung, das untere Ende der Röhre (1) an einer weiteren Anschlagoberfläche (AY2) der Haltekomponente (HO) zum Stillstand kommt;
wobei die Kanüle (KA) durch einen Kanülenentfernungstrennmechanismus von der Spritze (S) entfernbar ist,
**dadurch gekennzeichnet, dass** die Röhre (1) und die Hülse (3) betriebsfähig sind, um eine gemeinsame, längs verlaufende Mittelachse herum derart zu drehen, dass dickere Wandteile der Hülse (3) unmittelbar vor dem flexiblen Streifen (14) in der Röhre (1) zu liegen kommen und für die Entfernung der Kanüle (KA) aus der Spritze (S) eine Klemmwirkung auf den flexiblen Streifen (14) ausüben, wobei die Klemmwirkung einen inneren Absatz (15), der sich auf dem Inneren des flexiblen Streifens der Röhre (1) befindet, in eine Spur (SP) hinein, die um das vordere Ende des Injektionskörpers (SK) herumgeht, in deb Injektionskörper (SK) drückt und so ein Klemmen der Röhre (1) relativ zu dem Injektionskörper (SK) bereitstellt.

2. Vorrichtung nach Anspruch 1, **dadurch gekennzeichnet, dass** das Gehäuse (3) ferner Spuren (34) zum gegenseitigen Zusammenwirken mit den Eingriffskomponenten an mindestens einem Absatz (13) der Röhre (1) umfasst.

3. Verfahren zum Erhöhen der Sicherheit im Zusammenhang mit der Entfernung einer Kanüle (KA) aus einer Spritze durch die Vorrichtung nach Anspruch 1, **dadurch gekennzeichnet, dass** das Verfahren umfasst:
- Montieren einer Hülse (3) auf einer Röhre (1);
- Schieben der Hülse (3) und der Röhre (1) auf einen Injektionskörper (SK) der Spritze;
- Montieren einer Kanüle (KA) auf den Injektionskörper (SK);
- Schieben der Hülse (3) und der Röhre (1) zu einem vorderen Ende des Injektionskörpers (SK) hin derart, dass sich die Klemmwirkung drückt ein innerer Absatz (15) auf dem Inneren eines flexiblen Streifens der Röhre (1) befindet,
wobei der flexible Streifen (14) zwischen jeweils zwei Paaren angrenzender Schlitze einer Gruppe paralleler Längsschlitze, die aus zwei diametral angeordneten Paaren paralleler Schlitze (11A, 11B, 16A, 16B) besteht, angeordnet ist und in eine Spur (SP) einrastet, die um das vordere Ende des Injektionskörpers (SK) herum verläuft;
- Drehen der Röhre (1) hinsichtlich der Hülse (3) zum Verankern der Röhre (1) und der Hülse (3) auf dem Injektionskörper (SK) derart, dass dickere Wandteile der Hülse (3) unmittelbar vor dem flexiblen Streifen (14) in der Röhre (1) zu liegen kommen und eine Klemmwirkung auf den flexiblen Streifen (14) ausüben;
- Bewegen der Röhre (1), der Hülse (3) und der Spritze (S) in die Halteeinheit (HO) eines Kanülenentfernungstrennmechanismus derart, dass, während der Abwärtsverschiebung der Hülse (3) und der Röhre (1), die Hülse (3) an einer inneren ersten Anschlagoberfläche (AY1) des Halteelements (HO) zum Stillstand kommt und, während der weiteren Abwärtsverschiebung, das untere Ende der Röhre (1) an einer weiteren Anschlagoberfläche (AY2) des Halteelements (HO) zum Stillstand kommt; und
- Entfernen der Kanüle von dem Injektionskörper durch einen Kanülenentfernungstrennmechanismus.

4. Verfahren nach Anspruch 3, **dadurch gekennzeichnet, dass** die Klemmwirkung zwischen der Röhre (1) und dem Injektionskörper (SK) durch eine variierende Wandstärke der Hülse (3) erreicht wird.

## Revendications

1. Dispositif permettant d'augmenter la sécurité conjointement au retrait d'une canule (KA) d'un corps d'injection (SK) d'une seringue, le dispositif comprenant :
- un tube (1) ayant un groupe de fentes longitudinales parallèles constitué de deux paires diamétralement disposées de fentes parallèles (11A, 11B, 16A, 16B), dans lequel une bande flexible (14) est définie entre chacune des deux paires de fentes adjacentes du groupe de fentes longitudinales parallèles (11A, 11B, 16A, 16B) ;
- une enveloppe (3) montée sur le tube (1) et pourvue de deux évidements internes (31) s'étendant dans une direction longitudinale de l'enveloppe (3), dans lequel l'enveloppe (3) a une épaisseur de paroi variable dans la direction circonférentielle ; et
- un composant de maintien (HO) qui est capable de recevoir l'enveloppe (3) ainsi que le tube (1), dans lequel, lors d'un déplacement vers le bas de l'enveloppe (3) et du tube (1), l'enveloppe (3) vient s'appuyer contre une première surface de butée interne (AY1) du composant de maintien (HO) et, lors d'un déplacement ultérieur vers le bas, l'extrémité inférieure du tube (1) vient s'appuyer contre une autre surface de butée (AY2) du composant de maintien (HO) ;
dans lequel la canule (KA) peut être retirée de la seringue (S) par un mécanisme de séparation de retrait de canule,
**caractérisé en ce que** le tube (1) et l'enveloppe (3) sont capables de tourner autour d'un axe central commun s'étendant longitudinalement de telle sorte que des parties de paroi plus épaisses de l'enveloppe (3) se retrouvent directement devant la bande flexible (14) dans le tube (1) et exercent une action de serrage sur la bande flexible (14) pour le retrait de la canule (KA) de la seringue (S), dans lequel l'action de serrage presse un talon interne (15) situé sur l'intérieur de la bande flexible du tube (1) dans un rail (SP) contournant l'extrémité avant du corps d'injection (SK) dans le corps d'injection (SK), assurant ainsi le serrage du tube (1) par rapport au corps d'injection (SK).

2. Dispositif selon la revendication 1, **caractérisé en ce que** l'enveloppe (3) comprend en outre des rails (34) permettant de coopérer mutuellement avec les composants de mise en prise de l'au moins un talon (13) du tube (1).

3. Procédé d'augmentation de la sécurité conjointement au retrait d'une canule (KA) d'une seringue par le dispositif selon la revendication 1,
**caractérisé en ce que** le procédé comprend :
- le montage d'une enveloppe (3) sur un tube (1) ;
- le fait de pousser l'enveloppe (3) et le tube (1) sur un corps d'injection (SK) de la seringue ;
- le montage d'une canule (KA) sur le corps d'injection (SK) ;
- le fait de pousser l'enveloppe (3) et le tube (1) vers une extrémité avant du corps d'injection (SK), de telle sorte qu'un l'action de serrage appuie talon interne (15) situé sur l'intérieur d'une bande flexible du tube (1),
dans lequel la bande flexible (14) est définie entre chacune des deux paires de fentes adjacentes d'un groupe de fentes longitudinales parallèles constitué de deux paires diamétralement disposées de fentes parallèles (11A, 11B, 16A, 16B), s'enclenche dans un rail (SP) contournant l'extrémité avant du corps d'injection (SK) ;
- la rotation du tube (1) par rapport à l'enveloppe (3) pour ancrer le tube (1) et l'enveloppe (3) sur le corps d'injection (SK) de telle sorte que des parties de paroi plus épaisses de l'enveloppe (3) se retrouvent directement devant la bande flexible (14) dans le tube (1) et exercent une action de serrage sur la bande flexible (14) ;
- le déplacement du tube (1), de l'enveloppe (3) et de la seringue (S) dans l'unité de maintien (HO) d'un mécanisme de séparation de retrait de canule, de telle sorte que, lors d'un déplacement vers le bas de l'enveloppe (3) et du tube (1), l'enveloppe (3) vient s'appuyer sur une première surface de butée interne (AY1) du composant de maintien (HO) et, lors d'un déplacement ultérieur vers le bas, l'extrémité inférieure du tube (1) vient s'appuyer sur une autre surface de butée (AY2) du composant de maintien (HO) ; et
- le retrait de la canule du corps d'injection par un mécanisme de séparation de retrait de canule.

4. Procédé selon la revendication 3, **caractérisé en ce que** l'action de serrage entre le tube (1) et le corps d'injection (SK) est obtenue par une épaisseur de paroi variable de l'enveloppe (3).
